# EUROPEAN PATENT APPLICATION

(11) **EP 2 298 407 A1**
(43) Date of publication of application: **23.03.2011**
(21) Application number: 09738692.4
(22) Date of filing: 07.04.2009
(51) Int. Cl.: A61M 39/02, A61M 39/10, A61M 39/26, A61J 1/10

(54) **CONNECTOR ASSEMBLY**

(30) Priority: 02.05.2008 JP 2008120475
(71) Applicant: Terumo Kabushiki Kaisha, Shibuya-ku Tokyo 151-0072 (JP)
(72) Inventor: HIRANUMA, Takaaki, Nakakoma-gun Yamanashi 409-3853 (JP); TAKEMOTO, Masafumi, Nakakoma-gun Yamanashi 409-3853 (JP); OKABE, Hiromitsu, Tokyo 151-0072 (JP)
(74) Representative: TBK-Patent
(86) International application number: PCT/JP2009/057115
(87) International publication number: WO 2009/133755

(57) **Abstract**

A connector assembly is provided with a first connector equipped with a first connector body, a hollow needle supported on the inner side of the first connector body and having a side hole (53), and a first sealing member having a head which can be pierced through by the hollow needle; a second connector equipped with a second connector body and also with a second sealing member which can, when in a mounted state, be pierced through by the hollow needle; and a close-contact maintaining means which can, when in the mounted state, maintain that the head and the second sealing member are in close contact with each other. When the second connector becomes pulled out of the first connector, the head and the second sealing member are held in close contact with each other by the close-contact maintaining means until the hollow needle is pulled out of the second sealing member.

## Description

### Technical Field

The present invention relates to a connector assembly.

### Background Art

Normally, a medical agent which is dangerous if a medical care worker touches it by mistake, such as carcinostatics, immunosuppressants, etc. is contained in a powdery state in a vial container (medical agent container) having a mouth part sealed with a rubber stopper.

In taking the medical agent out of such a vial container, operations as follows are carried out.

First, the mouth part of the vial container and a mouth part of a syringe into which a dissolving liquid has been portioned out are connected to each other through a connector (see Patent Document 1). Next, in this connected condition, the dissolving liquid is injected from the syringe into the vial container. Then, the medical agent is uniformly dissolved in the dissolving liquid by such an operation as a pumping operation or shaking of the vial container. Subsequently, the dissolving liquid with the medical agent dissolved therein (hereinafter referred to as "liquid medical agent") is taken out into the syringe by suction.

The connector described in Patent Document 1 that is used for such operations includes a hollow needle having a sharp needle point at its distal end, a hub (connecting part) for supporting the hollow needle, and a cover member for covering the needle point. The cover member can be moved along the longitudinal direction of the hollow needle, and can be displaced into a first position for covering the needle point and a second position for letting the needle point exposed. Further, this connector is equipped with a stopper (safety latch) which inhibits the cover member from being unwillingly moved from the first position to the second position. The connector constituted in this fashion can be used, for example, in the condition where the hub is connected (mounted) to the syringe, the cover member is put into the second position by operating the stopper, and the rubber stopper of the vial container is pierced through by the hollow needle (this condition will hereinafter be referred to as "use condition"). In this use condition, the inside of the syringe and the inside of the vial container communicate with each other through the connector (hollow needle).

However, the connector described in Patent Document 1 has a problem in that if a force such as to pull the hollow needle out of the rubber stopper of the vial container acts in the use condition, the hollow needle would be easily pulled out of the rubber stopper of the vial container. In this case, the liquid medical agent may be scattered from the exposed needle point, and adhere to a medical care worker or the like, or the medical care worker may be punctured with the needle point by mistake. Thus, there has been a problem that the liquid medical agent cannot be transferred safely and assuredly through the connector.
Patent Document 1: Japanese Laid-Open Patent Publication No. 2005-522282 (PCT)

### Disclosure of Invention

It is an object of the present invention to provide a connector assembly in which a liquid can be transferred safely and assuredly from the first connector side to the second connector side or in the reverse direction.

In order to attain the above object, according to the present invention, there is provided a connector assembly including:
a first connector equipped with a first connector body tubular in shape, a hollow needle supported on an inner side of the first connector body and having an opening part opening at a distal end portion thereof, and a first sealing member formed of an elastic material and having a first to-be-pierced part which can be pierced through by the hollow needle;
a second connector equipped with a second connector body tubular in shape, and a second sealing member formed of an elastic material and having a second to-be-pierced part which, in a mounted state of being inserted in the first connector, is pierced through by the hollow needle;
locking means for connecting the first connector and the second connector to each other in the mounted state;
unlocking means for releasing connection of the first connector and the second connector by the locking means; and
close-contact maintaining means which, in the mounted state, maintains close contact between the first to-be-pierced part and the second to-be-pierced part;
   wherein in the mounted state, the first to-be-pierced part and the second to-be-pierced part are pierced through by the hollow needle while being in close contact with each other, the opening part of the hollow needle is located on a distal end side relative to the second to-be-pierced part and exposed to an inside of the second connector body, and a lumen of the hollow needle and a lumen of the second connector body communicate with each other through the opening part; and
   when the second connector is pulled out of the first connector, the close contact between the first to-be-pierced part and the second to-be-pierced part is maintained by the close-contact maintaining means until the opening part of the hollow needle comes to be located on the proximal end side relative to the second to-be-pierced part.

This ensures that, in the mounted state, the lumen of the hollow needle of the first connector and the lumen of the second connector body of the second connector communicate with each other. A liquid can be transferred from the first connector side to the second connector side or from the second connector side to the first connector side through the lumen of the hollow needle and the lumen of the second connector which thus communicate with each other.

In addition, in the mounted state, the connection between the first connector and the second connector by the locking means is maintained. This makes it possible to securely prevent the second connector from being unwillingly pulled out of the first connector. Accordingly, a liquid can be transferred safely through the connector assembly in the mounted state.

Further, in the mounted state, the close contact between the first sealing member of the first connector and the second sealing member of the second connector is maintained. This makes it possible to securely maintain the liquid-tightness (gas-tightness) of the lumen of the hollow needle and the lumen of the second connector body. Accordingly, a liquid passing through the lumens can be securely prevented from leaking out of the connector assembly in the mounted state.

In addition, when the second connector is pulled out of the first connector, the close contact between the first to-be-pierced part and the second to-be-pierced part is maintained by the close-contact maintaining means until the opening part of the hollow needle comes to be located on the proximal end side relative to the second to-be-pierced part. This ensures that, even while the second connector is being pulled out of the first connector, the liquid-tightness of the lumen of the hollow needle and the lumen of the second connector body is maintained, and, therefore, a liquid in these lumens can be securely prevented from leaking out of the connector assembly. Accordingly, the transfer of the liquid can be carried out safely by use of the connector assembly.

Further, in the connector assembly according to the present invention, preferably, the close-contact maintaining means has a biasing member which is disposed inside the first connector body and which biases the first to-be-pierced part in a distal direction.

This ensures that the close-contact maintaining means can be simple in constitution. In addition, the close contact between the first to-be-pierced part and the second to-be-pierced part can be maintained assuredly. Therefore, in that condition, a liquid can be transferred safely and assuredly from the first connector side to the second connector side or in the reverse direction.

Further, in the connector assembly according to the present invention, preferably, a sliding resistance generated between the hollow needle and the first to-be-pierced part when the second connector is pulled out of the first connector is smaller than a biasing force of the biasing member.

This makes it possible to prevent the first sealing member from being unwillingly moved by the biasing force of the biasing member upon releasing of a pressing force exerted on the first sealing member in the mounted state.

In addition, in the connector assembly according to the present invention, preferably, the close-contact maintaining means has a fitting member being ring-like in shape, being disposed at an outer peripheral portion of the second connector body and, when in the mounted state, being fitted to an inner peripheral portion of the first connector body.

This ensures that the close-contact maintaining means can be simple in constitution. Further, the close contact between the first to-be-pierced part and the second to-be-pierced part is maintained assuredly. In that condition, therefore, a liquid can be transferred safely and assuredly from the first connector side to the second connector side or in the reverse direction.

In addition, in the connector assembly according to the present invention, preferably, a sliding resistance generated between the inner peripheral portion of the first connector body and the fitting member when the second connector is pulled out of the first connector is greater than the biasing force of the biasing member.

This prevents securely the second connector from being unwillingly pushed out (caused to fly out of the first connector) by the biasing force of the biasing member. Accordingly, the close contact between the first sealing member and the second sealing member is maintained.

Further, in the connector assembly according to the present invention, preferably, the close-contact maintaining means has a plurality of ribs which are formed at an outer peripheral portion of the second connector body in a longitudinal direction thereof and which, in the mounted state, make contact with an inner peripheral portion of the first connector body.

This ensures that the close-contact maintaining means can be simple in constitution. In addition, the close contact between the first to-be-pierced part and the second to-be-pierced part is maintained assuredly. In that condition, therefore, a liquid can be transferred safely and assuredly from the first connector side to the second connector side or in the reverse direction.

Further, in the connector assembly according to the present invention, preferably, a sliding resistance generated between the inner peripheral portion of the first connector body and the plurality of ribs when the second connector is pulled out of the first connector is greater than the biasing force of the biasing member.

This makes it possible to securely prevent the second connector from being unwillingly pushed out by the biasing force of the biasing member when the connection between the first connector and the second connector has been released. Accordingly, in this situation, the close contact between the first sealing member and the second sealing member is maintained.

In addition, in the connector assembly according to the present invention, preferably, the close-contact maintaining means has a spiral groove formed in an inner peripheral portion of the first connector body, and a projection which is projectingly formed at an outer peripheral portion of the second connector body and which, in the mounted state, is inserted in the groove.

This ensures that the close-contact maintaining means is simple in constitution. Further, the close contact between the first to-be-pierced part and the second to-be-pierced part is maintained assuredly. In that condition, therefore, a liquid can be transferred safely and assuredly from the first connector side to the second connector side or in the reverse direction.

In addition, in the connector assembly according to the present invention, preferably,
an inner tube movable along an axial direction of the first connector body is disposed on the inner side of the first connector body; and
the close-contact maintaining means has an inner tube side engaging part provided at a distal end portion of the inner tube, and a second connector body side engaging part which is provided at an end portion of the second connector body and which, in the mounted state, is engaged with the inner tube side engaging part so as to compress the first to-be-pierced part and the second to-be-pierced part toward each other.

This ensures that the close-contact maintaining means can be simple in constitution. Further, the close contact between the first to-be-pierced part and the second to-be-pierced part is maintained assuredly. In that condition, therefore, a liquid can be transferred safely and assuredly from the first connector side to the second connector side and in the reverse direction.

In addition, in the connector assembly according to the present invention, preferably, the locking means comprises a first engaging part disposed on the first connector body so as to be movable in a radial direction of the first connector body, an elastic piece which is provided in the first connector body and which biases the first engaging part toward the inner side of the first connector body, and a second engaging part which is provided in the second connector body and which is engaged with the first engaging part.

This ensures that the first connector and the second connector are connected to each other, and the first connector and the second connector are prevented from being unwillingly released from each other.

Further, in the connector assembly according to the present invention, preferably, the fitting member is formed of an elastic material.

This makes it possible to securely maintain the close contact between the first to-be-pierced part and the second to-be-pierced part in the mounted state.

In addition, in the connector assembly according to the present invention, preferably,
the first connector body is equipped with a ring-shaped member which is ring-like in shape and which can be moved into a position where its axis is coaxial with the axis of the first connector body and a position where its axis is eccentric in relation to the axis of the first connector body;
the first engaging part is composed of a part of an edge portion of the ring-shaped member; and
the second engaging part is composed of an enlarged diameter part which is formed at an outer peripheral portion of the second connector body and at which the outside diameter of the outer peripheral portion is enlarged.

This ensures that the first connector and the second connector are connected to each other, and the first connector and the second connector are prevented from being unwillingly released from each other.

Further, in the connector assembly according to the present invention, preferably, the unlocking means is composed of a pressing part which is provided in the ring-shaped member and which presses the elastic piece against the biasing force of the elastic piece.

This ensures that at the time of releasing the connected state of the first connector and the second connector, the release can be easily achieved by pressing the elastic piece against the biasing force of the elastic piece, through the pressing part.

In addition, in the connector assembly according to the present invention, preferably, each of the first to-be-pierced part and the second to-be-pierced part is plate-line in shape and is so disposed that its thickness direction coincides with the axial direction.

This ensures that the first to-be-pierced part and the second to-be-pierced part can be collectively pierced through by the hollow needle in the mounted state.

Further, in the connector assembly according to the present invention, preferably, the first to-be-pierced part has a protuberance which is protuberant in the direction of the tip, and the second to-be-pierced part has a recess in which the protuberance is inserted into the mounted state.

This ensures that the area of close contact between an end surface of the first sealing member and an end surface of the second sealing member is enlarged, so that the liquid-tightness (gas-tightness) of a first flow path and a second flow path, particularly at the joint between the flow paths, can be maintained more securely. Accordingly, a liquid passing through these flow paths can be securely prevented from leaking out of the connector assembly in the assembled state.

In addition, the connector assembly according to the present invention, preferably, includes insertion depth restricting means for restricting a maximum depth of insertion of the second connector into the first connector in the mounted state.

This makes it possible to prevent the second connector from being excessively inserted into the first connector.

Further, in the connector assembly according to the present invention, preferably, the first connector is mounted to a mouth part of a syringe outer tube, the mouth part being provided in a tubular shape at a distal end portion of the syringe outer tube.

This ensures that a liquid can be transferred safely and assuredly from the first connector side to the second connector side or in the reverse direction.

In addition, in the connector assembly according to the present invention, preferably, the second connector is mounted to a mouth part of a liquid container capable of containing a liquid.

This ensures that a liquid can be transferred safely and assuredly from the first connector side to the second connector side or in the reverse direction.

### Brief Description of Drawings

FIG. 1 is an exploded side view of a connector assembly (first embodiment) according to the present invention;
FIG. 2 is a longitudinal sectional view illustrating a process until a first connector and a second connector in the connector assembly according to the present invention are brought into an assembled state;
FIG. 3 is a longitudinal sectional view illustrating the process until the first connector and the second connector in the connector assembly according to the present invention are brought into the assembled state;
FIG. 4 is a longitudinal sectional view illustrating the process until the first connector and the second connector in the connector assembly according to the present invention are brought into the assembled state;
FIG. 5 is a longitudinal sectional view illustrating a process until the connector assembly (in the assembled state) shown in FIG. 4 is brought into a disassembled state;
FIG. 6 is a longitudinal sectional view illustrating the process until the connector assembly (in the assembled state) shown in FIG. 4 is brought into the disassembled state;
FIG. 7 is a longitudinal sectional view illustrating the process until the connector assembly (in the assembled state) shown in FIG. 4 is brought into the disassembled state;
FIG. 8 is a longitudinal sectional view illustrating the first connector of the connector assembly (first embodiment) according to the present invention;
FIG. 9 is a longitudinal sectional view illustrating the second connector of the connector assembly (first embodiment) according to the present invention;
FIG. 10 is a side view illustrating a second connector of a connector assembly (second embodiment) according to the present invention;
FIG. 11 is a transverse sectional view illustrating a first connector body and a second connector body in a mounted state of the connector assembly (second embodiment) according to the present invention;
FIG. 12 is a partial longitudinal sectional view illustrating a first connector body and a second connector body in a connector assembly (third embodiment) according to the present invention;
FIG. 13 is a longitudinal sectional view illustrating a first connector and a second connector in a connector assembly (fourth embodiment) according to the present invention;
FIG. 14 is a longitudinal sectional view illustrating a second connector of a connector assembly (fifth embodiment) according to the present invention;
FIG. 15 shows longitudinal sectional views illustrating a process until a first sealing member and a second sealing member in a connector assembly (sixth embodiment) according to the present invention are brought into close contact with each other;
FIG. 16 shows longitudinal sectional views illustrating a process until a first sealing member and a second sealing member in a connector assembly (seventh embodiment) according to the present invention are brought into close contact with each other;
FIG. 17 shows longitudinal sectional views illustrating a process until a first sealing member and a second sealing member in a connector assembly (eighth embodiment) according to the present invention are brought into close contact with each other; and
FIG. 18 is a partial longitudinal sectional view illustrating a syringe which is mounted to the first connector shown in FIG. 8.

### Best Mode for Carrying Out the Invention

Now, the connector assembly according to the present invention will be described in detail below, based on preferred embodiments shown in the accompanying drawings.

### <First Embodiment>

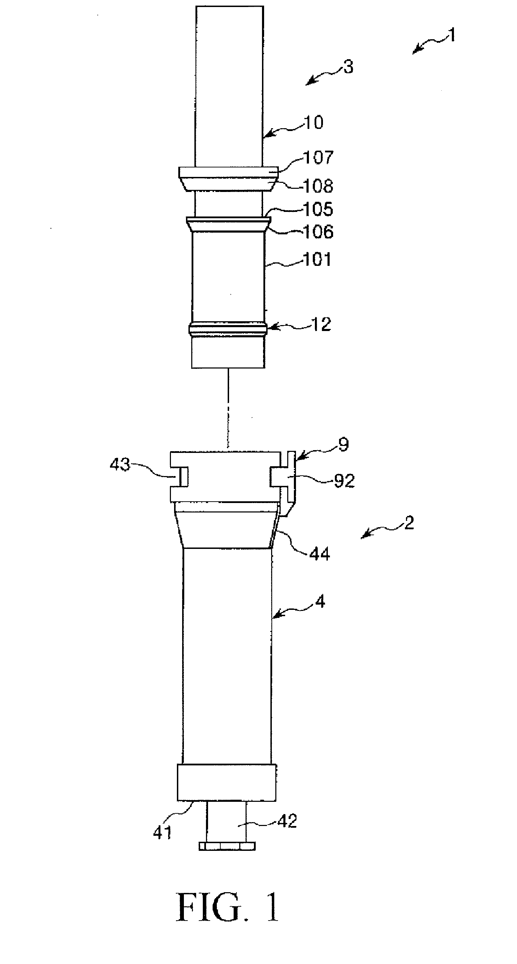

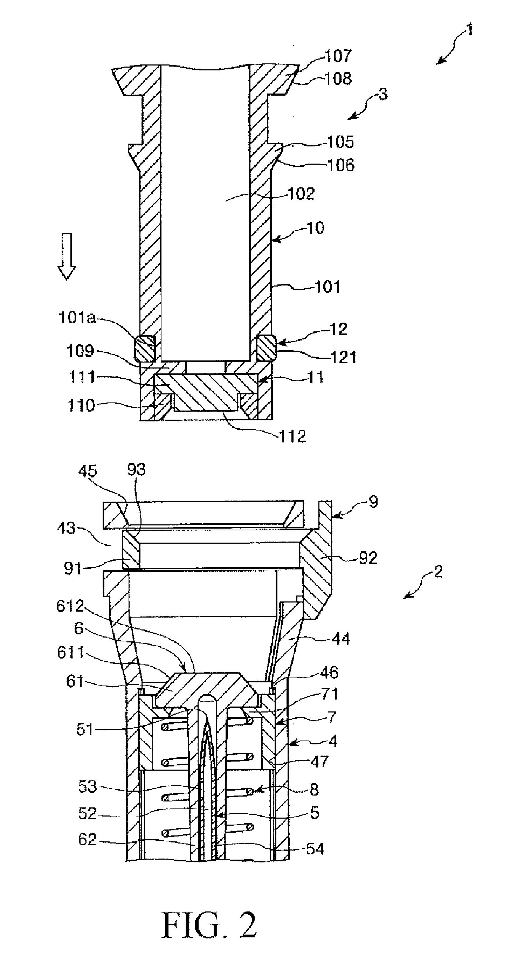

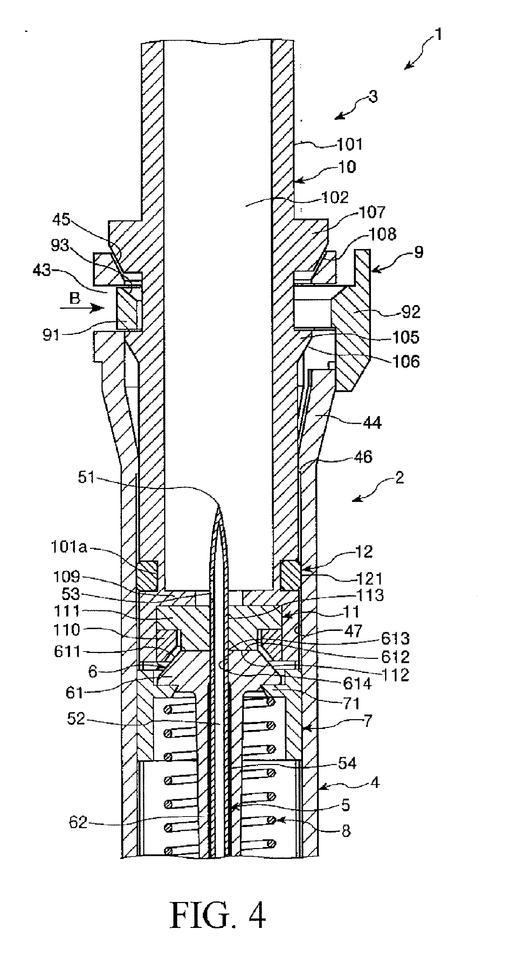

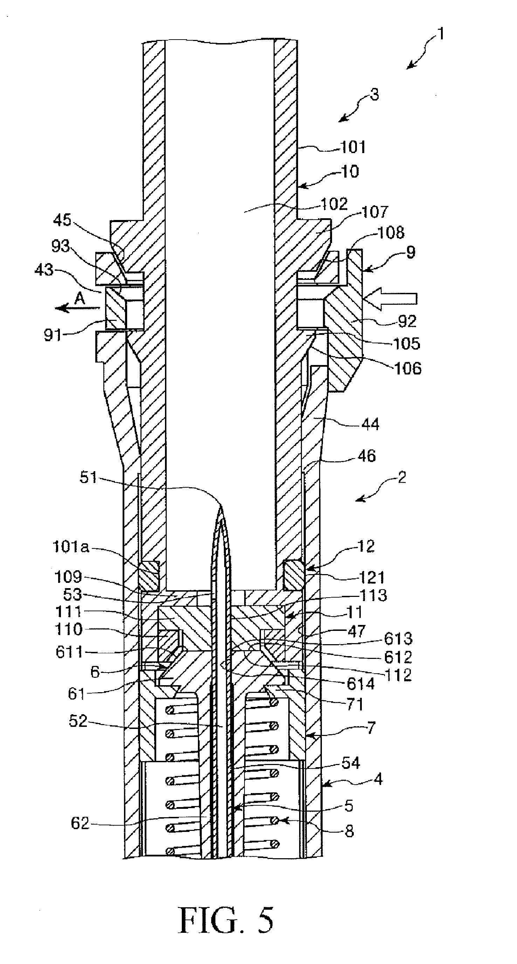

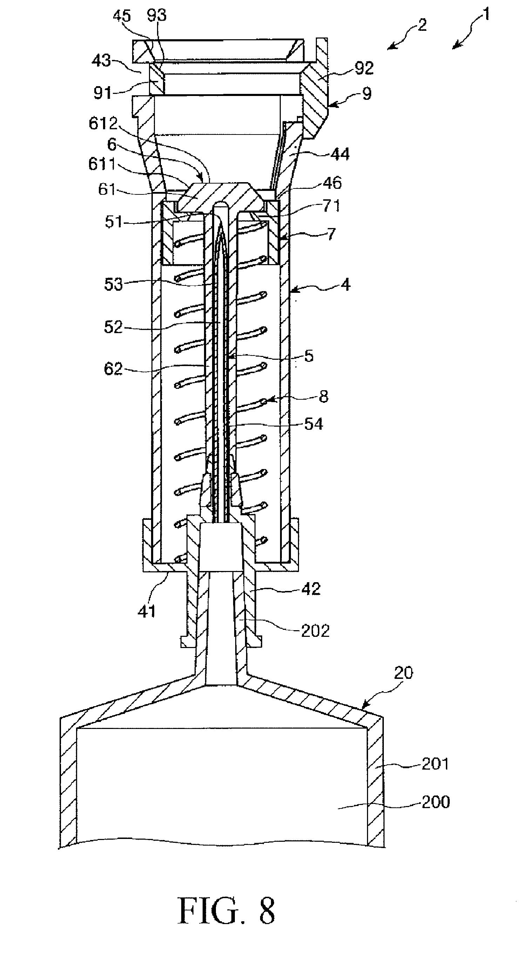

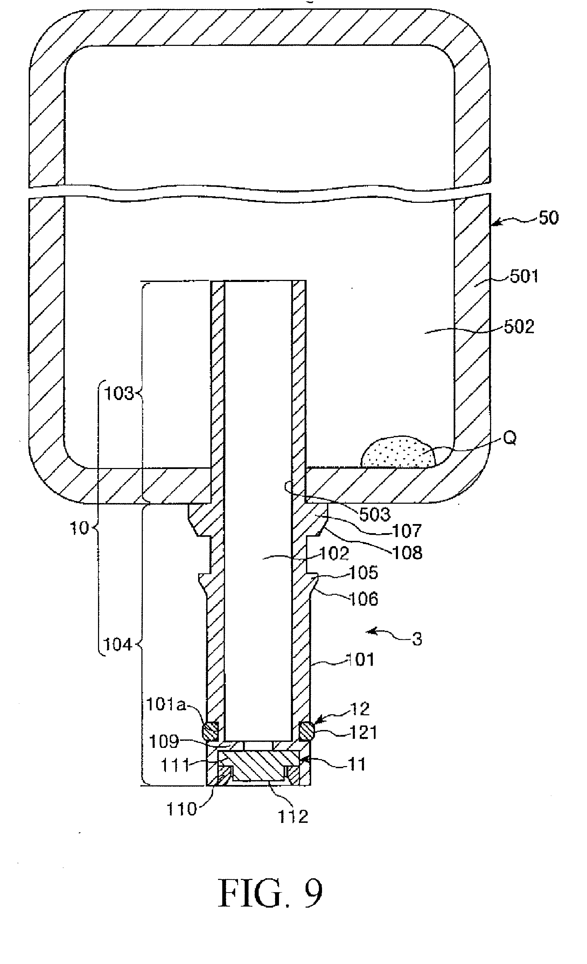

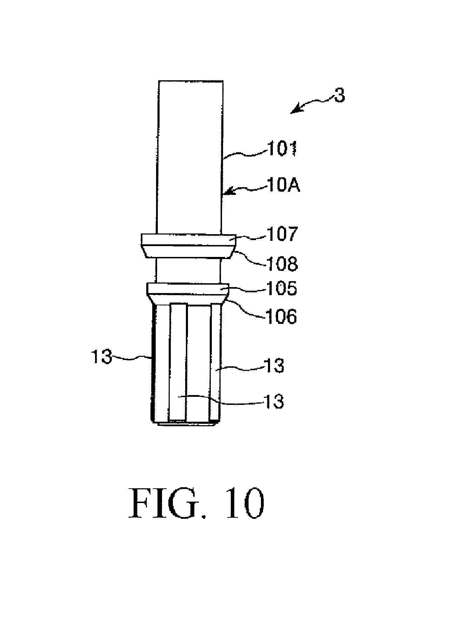

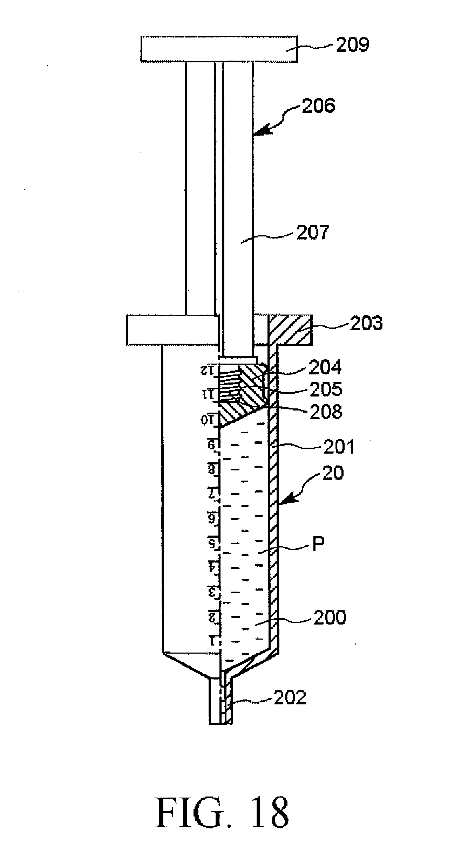

FIG. 1 is an exploded side view of a connector assembly (first embodiment) according to the present invention; FIGS. 2 to 4 are longitudinal sectional views illustrating a process until a first connector and a second connector in the connector assembly according to the present invention are brought into an assembled state (mounted state); FIGS. 5 to 7 are longitudinal sectional views illustrating a process until the connector assembly (in the assembled state) shown in FIG. 4 is brought into a disassembled state; FIG. 8 is a longitudinal sectional view illustrating the first connector of the connector assembly (first embodiment) according to the present invention; FIG. 9 is a longitudinal sectional view illustrating the second connector of the connector assembly (first embodiment) according to the present invention; and FIG. 18 is a partial longitudinal sectional view illustrating a syringe which is mounted to the first connector shown in FIG. 8. Incidentally, in the following description, for convenience of description, the upper side in FIGS. 1 to 9 (and in FIG. 10 and FIGS. 12 to 17, also) will be referred to as "distal end" and the lower side as "proximal end". Further, the upper side in FIG. 18 will be referred to as "proximal end" and the lower side as "distal end."

As shown in FIGS. 1 to 7, a connector assembly 1 has a first connector (female connector) 2 and a second connector (male connector) 3 which can be mounted to and dismounted from each other. As shown in FIG. 8, the first connector 2 is preliminarily mounted (connected) to a syringe (liquid container) 20. As shown in FIG. 9, the second connector 3 is mounted to a bag (liquid container) 50. The connector assembly 1, in an assembled state (the state shown in FIG. 4) in which these connectors are assembled to each other with the second connector 3 inserted into the first connector 2 from the distal end side of the latter, is used for transferring a liquid from the first connector 2 side to the second connector 3 side or in the reverse direction.

First, before describing the constitution of the connector assembly 1, description will be made of the constitution of the syringe 20 to which the first connector 2 of the connector assembly 1 is mounted and the constitution of the bag 50 to which the second connector 3 is mounted.

As shown in FIG. 18, the syringe 20 in the present embodiment includes an outer tube (syringe outer tube) 201, a gasket 204 slidable inside the outer tube 201, and a plunger (plunger rod) 206 operated to move the gasket 204 inside the outer tube 201 in the longitudinal direction (axial direction). The gasket 204 is connected to the distal end of the plunger 206.

The outer tube 201 is composed of a bottomed tubular member, and a reduced diameter part (mouth part) 202 reduced in diameter as compared with a barrel part of the outer tube 201 is formed projectingly and integrally at a central portion of a distal end-side bottom part of the outer tube 201. In addition, as shown in FIG. 8, the reduced diameter part 202 is a tubular part, and can be inserted into the first connector 2. This enables the syringe 20 (outer tube 201) and the first connector 2 to be connected to each other.

A flange (outer tube side flange) 203 is projectingly formed integrally at the outer periphery of the rear end of the outer tube 201.

Further, the outer tube 201 is provided with graduations for indicating the amount of liquid on its outer peripheral surface.

A material for the outer tube 201 includes various resins such as, for example, polyvinyl chloride, polyethylene, polypropylene, cyclic polyolefin, polystyrene, poly-(4-methylpentene-1), polycarbonate, acrylic resin, acrylonitrile-butadiene-styrene copolymer, polyester such as polyethylene terephthalate and polyethylene naphthalate, butadiene-styrene copolymer and polyamide (for example, nylon 6, nylon 6·6, nylon 6·10 and nylon 12). However, among them, such resins as polypropylene, cyclic polyolefin and polyester are preferable in that molding is easy and the water vapor permeability is low. It is to be noted that preferably the material for the outer tube 201 is substantially transparent in order to assure the visibility of the inside.

In the outer tube 201 as mentioned above, the gasket 204 formed of an elastic material is contained (inserted). The gasket 204 is provided with a plurality of (two) ring-shaped projections which are formed along the whole circumference, at its outer peripheral portion. The projections are slid in close contact with the inner peripheral surface of the outer tube 201, whereby liquid-tightness can be maintained more assuredly and an enhanced slidability can be contrived.

In addition, the gasket 204 is formed with a hollow part 205 opened in the rear end face thereof. In the hollow part 205, a head part 208 of the plunger 206 to be described later is screw-engaged (inserted). The inner surface of the hollow part 205 is formed with a female screw.

Although the material for the gasket 204 is not limited particularly, elastic materials such as various rubber materials such as, for example, natural rubber, butyl rubber, isoprene rubber, butadiene rubber, styrene-butadiene rubber and silicone rubber, various thermoplastic elastomers such as polyurethane-based, polyester-based, polyamide-based, olefin-based and styrene-based elastomers or mixtures of them can be used.

The plunger 206 has a bar-shaped main body part 207 which is cross-shaped in cross section.

The main body part 207 is provided with the head part (connection part) 208 on its distal end side. The head part 208 is inserted in the hollow part 205 of the gasket 204 and connected to the gasket 204. The head part 208 is provided at its outer periphery with a male screw which can be screw-engaged with the female screw at the inner surface of the hollow part 205. By putting the male screw and the female screw into screw engagement with each other, the gasket 204 and the plunger 206 are interconnected. Incidentally, the gasket 204 and the plunger 206 are not restricted to the constitution in which they are interconnected by screw engagement. There may also be adopted, for example, a constitution in which they are connected by fitting of a projection and a recess or the like, a constitution in which they are secured to each other by adhesion, fusing or the like, or a constitution in which they are integrally molded.

Further, the main body part 207 is provided at its rear end with a circular disk-shaped (plate-shaped) flange 209.

In addition, as the material constituting the plunger 206, materials identical to those mentioned above as material for the outer tube 201 can be used.

In the syringe 20 constituted as above, a space 200 surrounded by the outer tube 201 and the gasket 204 is preliminarily filled with a dissolving liquid P (e.g., physiological saline) for dissolving, for example, a powdery medical agent Q contained in the bag 50. The dissolving liquid P is supplied from the syringe 20 into the bag 50 through the connector assembly 1 in the assembled state, and the medical agent Q in the state of being dissolved in the dissolving liquid (liquid medical agent), is administered.

The bag 50 is for containing the powdery medical agent Q. The bag 50 is constituted by joining edge portions 501 of two flexible sheet materials to each other by, for example, fusing (heat fusing, microwave fusing, ultrasonic fusing or the like). In the bag 50, the medical agent Q can be contained in a space 502 surrounded by these sheet materials.

Further, the bag 50 is formed with a part where the edge portions 501 of the sheet materials are not fused to each other. This part serves as a mouth part 503 at the time of taking the medical agent Q out of the bag 50, when the second connector 3 is inserted therein and joined thereto. Incidentally, the method for the joining is not particularly limited, and examples of the method include a method by adhesion (adhesion with an adhesive or a solvent) and a method by fusing (heat fusing, microwave fusing, ultrasonic fusing or the like).

The bag 50 is flexible as mentioned above, and, for example, a soft resin material (elastic material) can be used to form the bag 50. The soft resin material is not particularly limited. The soft resin material may include polyolefin such as polyethylene, polypropylene, ethylenevinyl acetate copolymer, polyvinyl chloride, polybutadiene, polyamide, polyester, silicone, and the like. Among them, polybutadiene is especially preferred. Where polybutadiene is used as the material for the bag 50, it is superior in suitable flexibility, chemical resistance and anti-adsorption property of chemicals.

Incidentally, the medical agent Q is not specifically restricted. Examples of the medical agent Q include those medical agents which are dangerous if a medical care worker touches them by mistake, such as carcinostatics, immunosuppressants, etc., those which need dissolution when put to use, such as antibiotics, hemostatics, etc., those which need dilution, such as pediatric medical agents, etc., and those which are portioned out a plurality of times, such as vaccines, heparin, pediatric medical agents, etc. In addition, the medical agent Q is not limited to powdery medical agents but may be, for example, a liquid medical agent.

Now, the connector assembly 1 will be described below. As shown in FIGS. 1 to 7, the connector assembly 1 has the first connector 2 and the second connector 3.

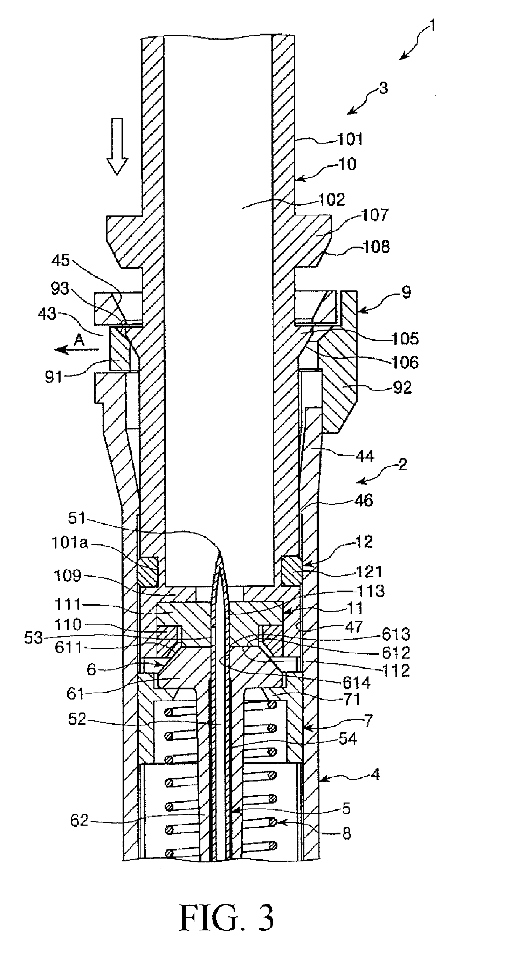

As shown in FIG. 8 (and in FIGS. 2 to 7, as well), the first connector 2 includes a cylindrical first connector body 4, a hollow needle 5 supported inside the first connector body 4, a first sealing member 6 disposed inside the first connector body 4 so as to be movable in the axial direction of the latter, an inner tube 7 moved together with the first sealing member 6, a coil spring 8 as a biasing member for biasing the first sealing member 6 in the distal direction, and a ring-shaped member 9 disposed on the first connector body 4 so as to be movable in the radial direction of the latter.
As shown in FIG. 8, the first connector body 4 is in the shape of a bottomed tube. A bottom part 41 of the first connector body 4 is provided with a tubular hub part 42 at its central portion, concentrically with the first connector body 4. The hub part 42 can support a proximal end portion of the hollow needle 5 on its distal end portion. In addition, the mouth part 202 of the syringe 20 can be inserted into a proximal end portion of the hub part 42. By this, the first connector 2 is mounted to the syringe 20, and the first connector 2 is used in this mounted state. Further, in the mounted state, the space 200 in the syringe 20 and the lumen (first flow path 52) of the hollow needle 5 communicate with each other through the hub part 42. This enables the dissolving liquid P to be supplied from the syringe 20 into the hollow needle 5.
In addition, the first connector body 4 is provided at its distal end opening part with a tapered part 45 the inside diameter of which increases gradually in the distal direction.
A wall part of the first connector body 4 is formed in its distal end portion with a groove 43 along the circumferential direction. The ring-shaped member 9 which is ring-like in shape is inserted into the groove 43.
As shown in FIGS. 2 to 7, the ring-shaped member 9 can be moved in the radial direction (the direction (left-right direction in the figures) perpendicular to the axis) of the first connector body 4. Specifically, the ring-shaped member 9 can be moved to a position (see FIGS. 5 to 7) in which its axis substantially coincides with the axis of (it is substantially coaxial with) the first connector boxy 4 and a position (see FIGS. 2 to 4) in which the axes are staggered from (eccentric to) each other.
As shown in FIG. 4, in the assembled state, the ring-shaped member 9 is so positioned as to be eccentric to the first connector body 4, and its first engaging part 91 (as a part of its edge portion) is engaged with a second engaging part 105 of the second connector 3 which will be described later. This results in that the first connector 2 and the second connector 3 are connected to each other, and the second connector 3 is prevented from being released from the first connector 2 unwillingly. Thus, in the connector assembly 1, the first engaging part 91 disposed in the first connector 2 and the second engaging part 105 disposed in the second connector 3 function, in the assembled state, as locking means for connecting the first connector and the second connector.
In addition, as shown in FIG. 5, with the first engaging part 91 retracted (separated) from the second engaging part 105 in the direction of arrow A in FIG. 5, the engagement between the first engaging part 91 and the second engaging part 105 is released. This results in that the second connector 3 can be pulled out of the first connector 2 (see FIGS. 5 to 7).
Thus, in the connector assembly 1 in the assembled state, when the ring-shaped member 9 is in the position such that its axis substantially coincides with the axis of the first connector body 4, the first engaging part 91 can be engaged with the second engaging part 105. This position can be referred to as "engagement position." Further, when the ring-shaped member 9 is in the position such that its axis is eccentric to the axis of the first connector body 4, the engagement between the first engaging part 91 and the second engaging part 105 is released. This position can be referred to as "disengagement position." Further, an operating piece (pressing part) 92 operated to press the ring-shaped member 9 is provided at an outer peripheral portion of the ring-shaped member 9. The operating piece 92 is biased by an elastic piece 44 composed of a part of the wall portion of the first connector body 4. The direction of biasing by the elastic piece 44 is the direction (the direction of arrow B in FIG. 4) in which the ring-shaped member 9 is moved from the disengagement position to the engagement position. In the assembled state shown in FIG. 4, the elastic piece 44 biases the first engaging part 91 in the direction of arrow B in the figure (toward the inner side of the first connector body) for engagement with the second engaging part 105. This ensures that, in the assembled state, the ring-shaped member 9 is prevented from being moved from the engagement position to the disengagement position (in the direction opposite to arrow B in FIG. 4) unwillingly. Therefore, the engaged state of the first engaging part 91 and the second engaging part 105 (the connected state of the first connector 2 and the second connector 3) is maintained. Consequently, the first connector 2 and the second connector 3 are securely prevented from being released from each other unwillingly.
In addition, for releasing the connected state of the first connector 2 and the second connector 3 attained by engagement between the first engaging part 91 and the second engaging part 105, the operating piece 92 of the ring-shaped member 9 is operated to press the elastic piece 44 against the biasing force of the elastic piece 44, whereby the connected state can be released (see FIG. 5). As shown in FIG. 5, with the operating piece 92 pressed, the first engaging part 91 is moved in the direction of arrow A in the figure. This releases the engagement between the first engaging part 91 and the second engaging part 105. In this disengaged state, the second connector 3 can be pulled out of the first connector 2 in the distal direction (see FIGS. 5 to 7). Thus, in the connector assembly 1, the operating piece 92 provided in the ring-shaped member 9 functions as unlocking means for releasing the connection between the first connector 2 and the second connector 3.
Further, the ring-shaped member 9 is provided at its inner peripheral portion with a first tapered part 93 of which the inside diameter gradually increases in the distal direction.
The materials constituting the first connector body 4 and the ring-shaped member 9 are not particularly limited. For example, such materials as mentioned above in relation to the outer tube 201 of the syringe 20 can be used.
As shown in FIG. 8, the hollow needle 5 formed of a metallic material is disposed on the axis of the first connector body 4. As has been mentioned above, the hollow needle 5 has its proximal end portion supported by the hub part 42 of the first connector body 4.
The hollow needle 5 is tubular in shape, and its lumen functions as a first flow path 52 through which the dissolving liquid P (liquid) can pass. In addition, the hollow needle 5 has its distal end closed, and is provided with a side hole (opening part) 53 which opens at a distal end portion of the wall part. The side hole 53 communicates with the first flow path 52.
The hollow needle 5 is formed at its distal end with a sharp needle point 51. As shown in FIGS. 3 to 6, the needle point 51 can pierce through the first sealing member 6 of the first connector 4 and a second sealing member (second to-be-pierced part) 11 of the second connector 3 which will be described later. As shown in FIG. 4, in the assembled state, the portion of the hollow needle 5 which ranges from the needle point 51 to the part formed with the side hole 53 is exposed (projected) to the inside of the second connector 3. This ensures that the lumen of the hollow needle 5 and the inside of the second connector 3 communicate with each other, namely, the first flow path 52 in the first connector 2 and a second flow path 102 in the second connector 3 to be described later communicate with each other, through the side hole 53 of the hollow needle 5.
As shown in FIG. 8, the first sealing member 6 is disposed inside the first connector body 4. The first sealing member 6 is moved along the axial direction of the first connector body 4, and can thereby be displaced into a sealing position for sealing the first flow path 52 on the distal end side of the hollow needle 5 (see FIGS. 2, 7 and 8) and a retracted position where it is retracted from the sealing position toward the proximal end side and is pierced through by the hollow needle 5 (see FIGS. 3 to 6). The first sealing member 6 is formed of an elastic material, which is not particularly restricted. For example, such materials as mentioned above in relation to the gasket 204 of the syringe 20 can be used.
The first sealing member 6 has a plate-shaped head part (first to-be-pierced part) 61, and a leg part 62 formed integrally on the proximal end side of the head part 61.
The head part 61 is a part which is circular disk-like in shape. The head part 61 is so provided that its thickness direction coincides with the axial direction of the hollow needle 5 (the axial direction of the first connector body 4). This ensures that, when the head part 61 is displaced along the axial direction of the hollow needle 5 toward the proximal end side, the head part 61 is pierced through by the needle point 51 of the hollow needle 5 easily and assuredly.
In addition, the head part 61 is formed at its distal end portion with a tapered part 611 the outside diameter of which gradually decreases in the distal direction.
The leg part 62 is a part which is tubular in shape. A proximal end portion of the leg part 62 is supported by the hub part 42 of the first connector body 4. The leg part 62 can be expanded and contracted in the longitudinal direction thereof. The leg part 62 is contracted when the head part 61 (the first sealing member 6) is located in the retracted position, and the leg part 62 is expanded by an elastic force of the head part 61 itself when the head part 61 is located in the sealing position. Thus, the leg part 62 can be said to be in charge of part of biasing means for biasing the first sealing member 6 in the direction (the distal direction) for displacement from the retracted position to the sealing position. In addition, the leg part 62 can contain the hollow needle 5 inside thereof when the head part 61 is located in the sealing position.
Further, in the connector assembly 1, a sliding resistance between an inner peripheral portion 614 of a piercing hole 613, formed in the head part 61 of the first sealing member 6 by the hollow needle 5, and an outer peripheral portion 54 of the hollow needle 5 at the time of pulling the second connector 3 out of the first connector 2 is set to be smaller than the biasing force of the coil spring 8. This ensures that, when the pressing force exerted on the head part 61 of the first sealing member 6 when the head part 61 is in the retracted position in the assembled state is released, the head part 61 can be returned into the sealing position by the biasing force of the coil spring 8. Incidentally, the method of setting the magnitude relationship between these forces is not specifically restricted. For example, a method can be adopted which is based on selection of the materials of the first sealing member 6 and the coil spring 8, adjustment of the thickness of the head part 61 of the first sealing member 6, adjustment of the wire diameter and the number of windings of the coil spring 8, adjustment of the outside diameter of the hollow needle 5, adjustment of the thickness of the leg part 62 of the first sealing member 6, or the like.
As shown in FIG. 8, in the inside of the first connector body 4, the tubular inner tube 7 is disposed which is movable along the axial direction of the first connector body 4. The inner tube 7 is formed at its inner peripheral portion with a reduced diameter part 71 having a reduced inside diameter. The reduced diameter part 71 is fixed to a proximal end face of the head part 61 of the first sealing member 6 by, for example, adhesion (adhesion with an adhesive or a solvent). This ensures that the inner tube 7 can be displaced together with the first sealing member 6 when the first sealing member 6 is displaced (see FIGS. 2 to 7). In addition, when the first sealing member 6 is located in the sealing position, the inner tube 7 makes contact with a stepped part 46 formed at an inner peripheral portion 47 of the first connector body 4, to be thereby restrained from moving in the distal direction. The material constituting the inner tube 7 is not specifically restricted. For example, such materials as above-mentioned in the description of the outer tube 201 of the syringe 20 can be used.
The coil spring 8 formed of a metallic material such as stainless steel is disposed inside the first connector body 4. The coil spring 8 is arranged on the outer periphery side of the leg part 62 of the first sealing member 6, and is penetrated by the leg part 62. In addition, in a compressed state of the coil spring 8, its distal end is in abutment on the reduced diameter part 71 of the inner tube 7, and its proximal end is in abutment on the bottom part 41 of the first connector body 4. This ensures that the first sealing member 6 can be assuredly biased in the distal direction. Incidentally, as the biasing means, a cylindrical or bellows-like rubber member may be used in place of the coil spring 8.
As shown in FIG. 4, when the second sealing member 11 of the second connector 3 presses the first sealing member 6 against the biasing force of the coil spring 8 in the assembled state, the head part 61 of the first sealing member 6 is biased in the distal direction by the coil spring 8, so that it is pressed against the second sealing member 11. This maintains the close contact between the first sealing member 6 and the second sealing member 11. Thus, in the connector assembly 1, the coil spring 8 functions as close-contact maintaining means which, in the assembled state, maintains the close contact between the first sealing member 6 and the second sealing member 11.
As shown in FIG. 9 (and in FIGS 2 to 7, as well), the second connector 3 includes a second connector body 10 which is tubular in shape, the second sealing member 11 provided in the second connector body 10, and a fitting member 12 mounted to an outer peripheral portion 101 of the second connector body 10.
The second connector body 10 is a member which is tubular in shape. A lumen of the second connector body 10 functions as the second flow path 102 permitting a liquid to pass therethrough. As shown in FIG. 9, the second connector body 10 has an intermediate portion joined to the bag 50, and can be divided into an inserted part 103 inserted in the bag 50, and an exposed part (protruded part) 104 exposed (protruded) from the bag 50.
The exposed part 104 of the second connector body 10 is provided with the second engaging part 105 at its outer peripheral portion 101. The second engaging part 105 is composed of an enlarged diameter part enlarged in outside diameter. As above-mentioned, the second engaging part 105 is a part which is engaged with the first engaging part 91 of the ring-shaped member 9 of the first connector 2 in the assembled state. The second engaging part 105 is provided at its proximal end portion with a second tapered part 106 of which the outside diameter gradually decreases in the proximal direction.
At the time of inserting the second connector 3 into the first connector 2 to obtain the assembled state, the cylindrical shape of the second connector body 10 ensures that the second connector 3 can be inserted at any of a plurality of rotational angles about the axis thereof in relation to the first connector 2. In addition, irrespectively of the rotational angle at which the second connector 3 is inserted into the first connector 2, the second engaging part 105 of the second connector 3 is assuredly engaged with the first engaging part 91 of the ring-shaped member 9 of the first connector 2 (see FIG. 4).
Further, at the outer peripheral portion 101 of the exposed part 104 of the second connector body 10, an enlarged diameter part (insertion depth restricting means) 107 enlarged in outside diameter is formed on the distal end side relative to the second engaging part 105. The enlarged diameter part 107 is provided at its proximal end portion with a tapered part 108 of which the outside diameter gradually decreases in the proximal direction.
As shown in FIGS. 2 to 4, at the time of inserting the second connector 3 into the first connector 2, the tapered part 108 of the enlarged diameter part 107 of the second connector 3 (the second connector body 10) can be abutted on the tapered part 45 of the first connector 2 (the first connector body 4), at an insertion limit (in the assembled state) of the second connector 3. This ensures that the maximum depth of insertion of the second connector 3 into the first connector 2 can be restricted, and, therefore, the second connector 3 can be prevented from being inserted into the first connector 2 in an excessive extent. In addition, by inserting the second connector 3 into the first connector 2 until the enlarged diameter part 107 (the tapered part 108) of the second connector 3 comes into abutment on the tapered part 45 of the first connector 2, the first engaging part 91 of the first connector 2 and the second engaging part 105 of the second connector 3 can be engaged with each other (see FIG. 4). Further, along with this, the side hole 53 of the hollow needle 5 of the first connector 2 opens into the inside of the second connector 3, whereby the first flow path 52 and the second flow path 102 are made to communicate with each other (see FIG. 4).
The material constituting the second connector body 10 is not particularly limited. For example, such materials as mentioned above in the description of the outer tube 201 of the syringe 20 can be used.
As shown in FIG. 9, the second sealing member 11 is inserted (sealed) in a proximal end portion of the second connector body 10. The second sealing member 11 can seal the second flow path 102 at a proximal end portion of the second flow path 102. In addition, the second sealing member 11 is, in the assembled state, pierced through by the hollow needle 5 of the first connector 2 (see FIG. 4). The second sealing member 11 is formed of an elastic material, which is not specifically restricted. For example, such materials as mentioned above in the description of the gasket 204 of the syringe 20 can be used.
The second sealing member 11 is a member which is circular disk-like (plate-like) in shape. The second sealing member 11 is so provided that its thickness direction coincides with the axial direction of the second connector body 10. This ensures that the second sealing member 11 is pierced through by the needle point 51 of the hollow needle 5 easily and assuredly when the connector assembly 1 is brought into the assembled state.
Further, the second sealing member 11 is provided at its outer peripheral portion with a flange part 111 enlarged in outside diameter. The flange part 111 is clamped on an inner peripheral portion of the second connector body 10 between an inside enlarged diameter part 109 enlarged in inside diameter and a ring-shaped pressed-in member 110 pressed into the second connector body 10 from the proximal end side of the latter. This ensures that the second sealing member 11 can be prevented from being released from the second connector body 10.
As shown in FIG. 9 (and in FIG. 1, as well), the ring-shaped fitting member 12 is mounted onto the portion of the exposed part 104 of the second connector body 10 which is on the proximal end side relative to the second engaging part 105. The fitting member 12 is mounted to the second connector body 10 by being inserted in a ring-shaped recess 101a formed in the outer peripheral portion 101 of the second connector body 10 along the circumferential direction of the latter. In addition, in a disassembled state (the state shown in FIGS. 1 and 9) of the connector assembly 1, an outer peripheral portion 121 of the fitting member 12 is protruding outward relative to the outer peripheral portion 101 of the second connector body 10. Specifically, the outside diameter of the fitting member 12, in the disassembled state, is greater than the outside diameter of the outer peripheral portion 101 of the second connector body 10. The fitting member 12 is formed of an elastic material, which is not specifically restricted. For example, such materials as mentioned above in the description of the gasket 204 of the syringe 20 can be used.
As shown in FIGS. 2 to 7, the fitting member 12 is compressed on the inner peripheral portion 47 of the first connector body 4 toward the inner side, in the process from the insertion of the second connector 3 into the first connector 2 to the pulling-out of the second connector 3 through the assembled state. This ensures that the fitting member 12 is assuredly fitted to the inner peripheral portion 47 of the first connector body 4. In addition, in the connector assembly 1, a sliding resistance generated between the outer peripheral portion 121 of the fitting member 12 and the inner peripheral portion 47 of the first connector body 4 when the second connector 3 is pulled out of the first connector 2 is set to be greater than the biasing force of the coil spring 8. The method for the setting is not particularly limited. For example, a method can be adopted which is based on selection of the materials of the fitting member 12 and the coil spring 8, adjustment of the wire diameter and/or the number of windings of the coil spring 8, adjustment of the inside diameter and the outside diameter and the thickness of the fitting member 12, adjustment of the outside diameter of the ring-shaped recess 101a, or the like.
Like the coil spring 8, the fitting member 12 as above-mentioned functions as close-contact maintaining means which, in the assembled state, maintains the close contact between the first sealing member 6 and the second sealing member 11, as will be described later. Further, the fitting member 12 can be said to be a member which receives the biasing force of the coil spring 8, in the second connector 3.
Now, the states of the first connector 2 and the second connector 3 during the process in which the connector assembly 1 is brought from the disassembled state to the assembled state and then again to the disassembled state will be described below, referring to FIGS. 2 to 7. [1] From disassembled state to assembled state (see FIGS. 2 to 4)

As shown in FIG. 2, the second connector 3 in the disassembled state is made to approach a distal end portion of the first connector 2, with its proximal end side first. In the disassembled state, the first connector 2 has the first sealing member 6 located in the sealing position, and the hollow needle 5 is contained in the first sealing member 6. This keeps the first flow path 52 in a closed state.

As shown in FIG. 3, as the second connector 3 is inserted into the first connector 2, first, a distal end face 612 of the first sealing member 6 located in the sealing position of the first connector 2 and a proximal end face 112 of the second sealing member 11 of the second connector 3 abut on each other. As the second connector 3 is inserted further into the first connector 2, the second sealing member 11 presses the first sealing member 6 in the proximal direction, against the biasing force of the coil spring 8 of the first connector 2. This causes the first sealing member 6 to start moving from the sealing position toward the retracted position. In this instance, the first sealing member 6 and the second sealing member 11 are sequentially pierced through by the hollow needle 5.

Then, the fitting member 12 and the second engaging part 105 of the second connector 3 are gradually inserted, in this order, into the first connector body 4 of the first connector 2. The fitting member 12 slides on the inner peripheral portion 47 of the first connector body 4 while being compressed by the inner peripheral portion 47 (see FIG. 3).

In addition, of the second engaging part 105 of the second connector 3, the second tapered part 106 abuts on the first tapered part 93 of the first engaging part 91 of the ring-shaped member 9 of the first connector 2. Attendant on the movement of the second connector 3 in the proximal direction, the first tapered part 93 of the first engaging part 91 is pressed by the second tapered part 106 in the direction of arrow A in FIG. 3 (see FIG. 3). In this instance, the ring-shaped member 9 is moved in the direction of arrow A in FIG. 3 against the biasing force of the elastic piece 44 of the first connector body 4.

As shown in FIG. 4, the second connector 3 is inserted into the first connector 2 up to a position where its enlarged diameter part 107 makes contact with, or is about to make contact with, the tapered part 45 of the first connector body 4, and the movement of the second connector 3 in the proximal direction is stopped. In this instance, the first engaging part 91 can ride over the second engaging part 105, and the ring-shaped member 9 is moved in the direction of arrow B in FIG. 4 by the biasing force (restoring force) of the elastic piece 44 of the first connector body 4. This ensures that the first engaging part 91 and the second engaging part 105 are engaged with each other, and the first connector 2 and the second connector 3 are connected to each other. As a result, the connection between the first connector 2 and the second connector 3 cannot be released unless the operating piece 92 of the ring-shaped member 9 is operated by depressing. Further, a click feeling is obtained when the first engaging part 91 rides over the second engaging part 105. This permits the user to grasp that the first engaging part 91 and the second engaging part 105 have engaged with each other.

In addition, the first sealing member 6 is moved (pressed) toward the proximal end side further than the state shown in FIG. 3, to be located in the retracted position. In this position, the first sealing member 6 is pierced through by the hollow needle 5 together with the second sealing member 11. Of the hollow needle 5 having pierced through these sealing members 6, the portion ranging from the needle point 51 to the part formed with the side hole 53 protrudes into the inside of the second connector body 10, and the side hole 53 opens toward the inside of the second connector body 10. Consequently, the first flow path 52 and the second flow path 102 communicate with each other through the side hole 53.

Further, even when the first sealing member 6 is located in the retracted position, the condition of close contact between its distal end face 612 and the proximal end face 112 of the second sealing member 11 is maintained by the biasing force of the coil spring 8.

In such an assembled state, the first flow path 52 in the first connector 2 and the second flow path 102 in the second connector 3 communicate with each other, as above-mentioned. Through the first flow path 52 and the second flow path 102 thus communicating with each other, the dissolving liquid P can be assuredly transferred from the first connector 2 side to the second connector 3 side, or from the second connector 3 side to the first connector 2 side.

In addition, in the assembled state, the first engaging part 91 of the first connector 2 and the second engaging part 105 of the second connector 3 remain in engagement with each other. This ensures that pulling of the second connector 3 out of the first connector 2, or unwilling disassembly of the connector assembly 1 in the assembled state, can be securely prevented from occurring. Consequently, the dissolving liquid P can be transferred safely through the connector assembly 1.

Further, in the assembled state, the close contact between the first sealing member 6 of the first connector 2 and the second sealing member 11 of the second connector 3 is maintained. This ensures that the liquid-tightness (gas-tightness) of the first flow path 52 and the second flow path 102, particularly in the vicinity of the joint between these flow paths, can be securely maintained. Accordingly, the dissolving liquid P passing through these flow paths is securely prevented from leaking out of the connector assembly 1 in the assembled state.

### [2] From assembled state to disassembled state again (see FIGS. 4 to 7)

When the operating piece 92 of the ring-shaped member 9 of the first connector 2 is operated by pressing as shown in FIG. 5, starting from the condition shown in FIG. 4, the first engaging part 91 of the first connector 2 is moved in the direction of arrow A in FIG. 5, to be spaced from the second engaging part 105 of the second connector 3. As a result, the engagement between the first engaging part 91 and the second engaging part 105 is released, in other words, the connection between the first connector 2 and the second connector 3 is released. This ensures that the second connector 3 can be moved in the distal direction, and an operation of pulling out the second connector 3 can be carried out.

In addition, in this instance, the coil spring 8 of the first connector 2 presses the second sealing member 11 through the first sealing member 6 by its biasing force, in the manner of attempting to push out the second connector 3 in the distal direction. Since the sliding resistance between the outer peripheral portion 121 of the fitting member 12 and the inner peripheral portion 47 of the first connector body 4 is greater than the biasing force of the coil spring 8 as described above, however, the second connector 3 is securely prevented from being unwillingly pushed out (from flying out of the first connector 2) under the biasing force of the coil spring 8. Accordingly, the close contact between the first sealing member 6 and the second sealing member 11 is maintained.

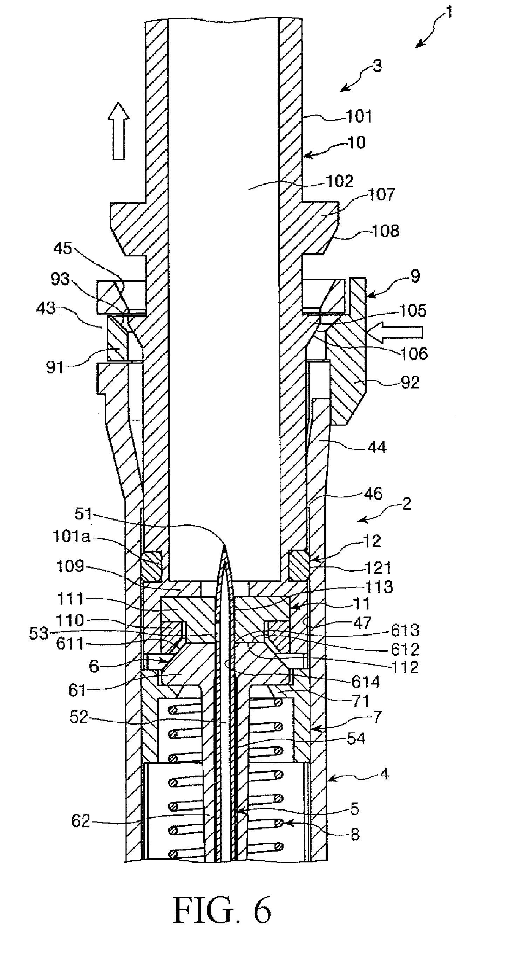

As shown in FIG. 6, when the second connector 3 is pulled in the distal direction relative to the first connector 2, starting from the condition shown in FIG. 5, the second connector 3 is moved in that direction. In this instance, since the sliding resistance between the inner peripheral portion 614 of the piercing hole 613 in the first sealing member 6 and the outer peripheral portion 54 of the hollow needle 5 is smaller than the biasing force of the coil spring 8 as above-mentioned, the first sealing member is moved toward the sealing position by the biasing force of the coil spring 8. In addition, the fitting force (sliding resistance) between the fitting member 12 and the inner peripheral portion 47 of the first connector body 4 at the time of pulling out the second connector 3 ensures that the moving speed of the first sealing member 6 is approximately equal to the moving speed of the second connector 3. Accordingly, the first sealing member 6 can be moved while making close contact with the second sealing member 11.

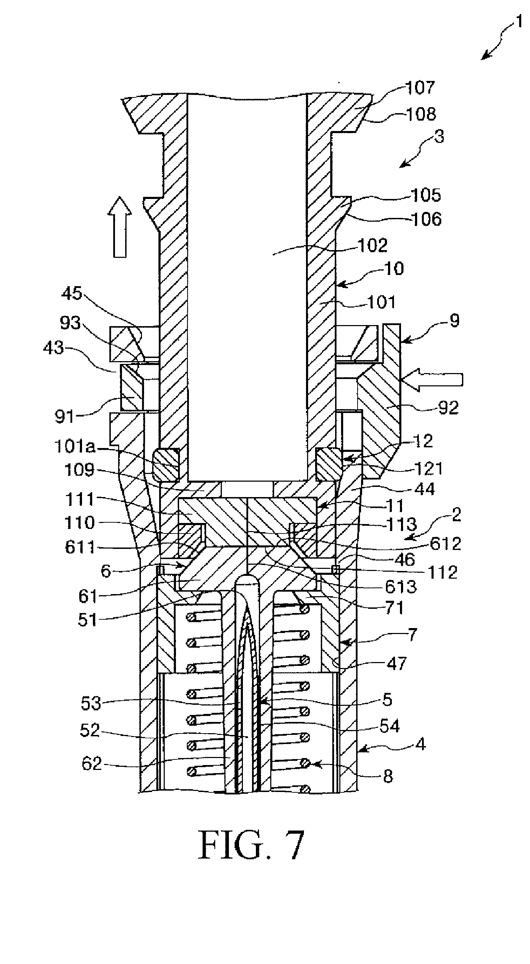

When the second connector 3 is further pulled in the distal direction relative to the first connector 2 as shown in FIG. 7, starting from the condition shown in FIG. 6, the first sealing member 6 is moved while making close contact with the second sealing member 11 in the same manner as above-mentioned, to return to the sealing position. In this instance, the side hole 53 of the hollow needle 5 is located on the proximal end side relative to the second sealing member 11, and is contained in the first sealing member 6. Further, the piercing hole 613 in the first sealing member 6 and a piercing hole 113 in the second sealing member 11 are respectively closed by their self-closing properties. Consequently, the first flow path 52 and the second flow path 102 are respectively closed.

When the second connector 3 is further pulled in the distal direction, the second connector 3 is pulled out of the first connector 2.

Thus, in the connector assembly 1, in the case of pulling the second connector 3 out of the first connector 2, the close contact of the first sealing member 6 with the second sealing member 11 is maintained until the first sealing member 6 is returned from the retracted position to the sealing position. This ensures that, even during disassembly of the connector assembly 1 in the assembled state, the liquid-tightness of the first flow path 52 and the second flow path 102 is maintained, and, therefore, the liquid medical agent (liquid) in these flow paths is securely prevented from leaking out of the connector assembly 1. Accordingly, the liquid medical agent can be safely transferred using the connector assembly 1.

Now, an example of the method of using the connector assembly 1 will be described below.

First, the first connector 2 mounted to the syringe 20 and the second connector 3 mounted to the bag 50 are prepared.

The first connector 2 and the second connector 3 are brought close to each other (see FIG. 1), and the second connector 3 is inserted into the first connector 2, to put them into the assembled state (see FIG. 4).

Next, the plunger 206 of the syringe 20 is pushed in the distal direction. This results in that the dissolving liquid P in the syringe 20 flows into the bag 50 through the connector assembly 1. Then, the bag 50 is shaken. This causes the medical agent Q to be uniformly dissolved in the dissolving liquid P.

Subsequently, the plunger 206 of the syringe 20 is pulled in the proximal direction, to suck the liquid medical agent into the syringe 20.

Next, the operating piece 92 of the first connector 2 is operated by depressing. This ensures that the connection between the first connector 2 and the second connector 3 is released. Then, the second connector 3 is pulled out of the first connector 2.

Subsequently, the first connector 2 is dismounted from the syringe 20, and the liquid medical agent is administered from the syringe 20.

### <Second Embodiment>

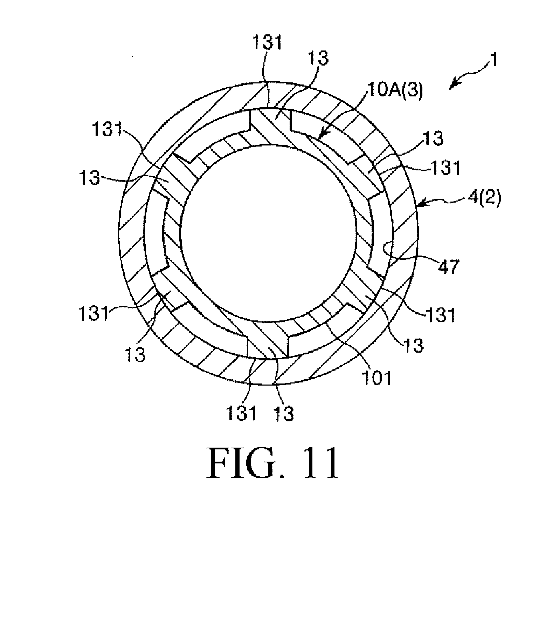

FIG. 10 is a side view illustrating a second connector in a connector assembly (second embodiment) according to the present invention, and FIG. 11 is a cross sectional view illustrating a first connector body and a second connector body in an assembled state in the connector assembly (second embodiment) of the present invention.

Now, the second embodiment of the connector assembly according to the present invention will be described below, referring to these figures. The following description will be centered on the difference of the present embodiment from the above-described embodiment, and descriptions of the same items as above will be omitted.

The present embodiment is the same as the above-described first embodiment, except for a difference in the constitution of the second connector body.

A second connector body 10A shown in FIGS. 10 and 11 is integrally provided with six ribs 13 formed along the longitudinal direction at its outer peripheral portion 101. As shown in FIG. 11, top portions 131 of the ribs are in contact with an inner peripheral portion 47 of the first connector body 4 in the assembled state. In addition, the ribs 13 are arranged at regular angular intervals along the circumferential direction of the outer peripheral portion 101 of the second connector body 10A.

In the connector assembly 1 in this embodiment, a sliding resistance between the inner peripheral portion 47 of the first connector body 4 and all the ribs 13 at the time of pulling the second connector 3 out of the first connector 2 is greater than a biasing force of a coil spring 8. Such a magnitude relationship between the forces ensures that, when the connection between the first connector 2 and the second connector 3 is released by operating an operating piece 92 of a ring-shaped member 9 of the first connector 2 by depressing, the second connector 3 is securely prevented from being unwillingly pushed out by the biasing force of the coil spring 8. Consequently, close contact between a first sealing member 6 and a second sealing member 11 is maintained. Thus, the ribs 13 function as close-contact maintaining means, like the fitting member 12 in the above-described first embodiment. In addition, a sliding resistance of the ribs 13 relative to the inner peripheral portion 47 of the first connector body 4 at the time of pulling out the second connector 3 permits the first sealing member 6 to be moved at a velocity comparable to that of the second connector 3. This enables the first sealing member 6 to move while making close contact with the second sealing member 11.

Thus, in the connector assembly 1 in this embodiment, at the time of pulling the second connector 3 out of the first connector 2, the close contact of the first sealing member 6 with the second sealing member 11 is maintained until it is returned from a retracted position to a sealing position. This ensures that liquid-tightness of a first flow path 52 and a second flow path 102 is maintained, and, therefore, a liquid medical agent in these flow paths is securely prevented from leaking out of the connector assembly 1.

Incidentally, while the number of the ribs formed is six in the present embodiment, this number is not limitative. For example, the number of the ribs may be two, three, four, five, or seven or more.

### <Third Embodiment>

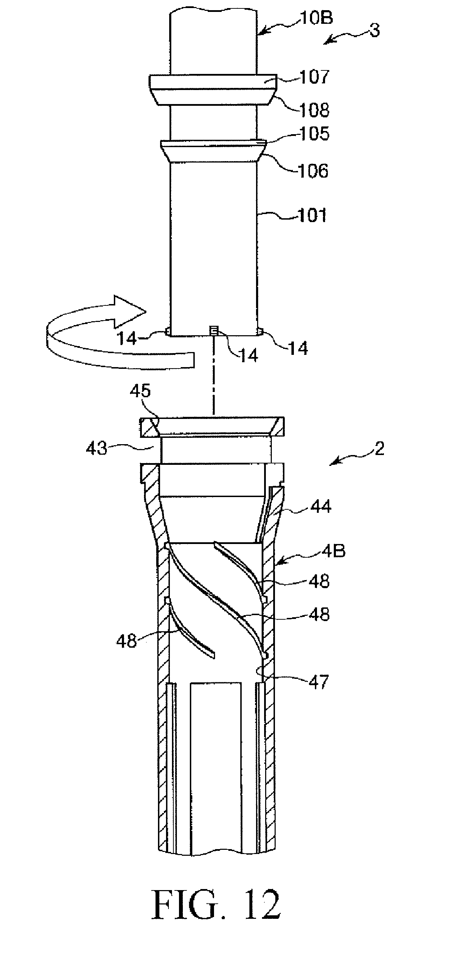

FIG. 12 is a partial longitudinal sectional view illustrating a first connector body and a second connector body in a connector assembly (third embodiment) according to the present invention.

Now, the third embodiment of the connector assembly according to the present invention will be described below, referring to the figure. The following description will be centered on a difference from the above-described embodiments, and descriptions of the same items as above will be omitted.

The present embodiment is the same as the above-described first embodiment, except for differences in the constitutions of the first connector body and the second connector body.

As shown in FIG. 12, a first connector body 4B is formed with four spiral grooves 48 in its inner peripheral portion 47. In addition, a second connector body 10B is projectingly provided with four projections 14 at a distal end portion of an outer peripheral portion 101 thereof. At the time of obtaining an assembled state, the second connector body 10B is rotated relative to the first connector body 4B about the axis thereof, whereby an operation of connecting the first connector 2 and the second connector 3 to each other can be performed. In this case, each of the projections 14 is inserted in each of the grooves 48, and is moved along the groove 48.

In the connector assembly 1 in the present embodiment, the projections 14 are moved along the grooves 48 as above-mentioned. When the connection between the first connector 2 and the second connector 3 is released by operating an operating piece 92 of a ring-shaped member 9 of the first connector 2 in the assembled state by depressing, a biasing force of a coil spring 8 securely prevents the second connector 3 from being pushed out unwillingly. Accordingly, close contact between a first sealing member 6 and a second sealing member 11 is maintained. Thus, the projections 14 and the grooves 48 function as close-contact maintaining means, like the fitting member 12 in the above-described first embodiment. Further, also in this embodiment, the first sealing member 6 can be moved at a velocity comparable to that of the second connector 3, like in the first embodiment described above. This permits the first sealing member 6 to move while making contact with the second sealing member 11 until it is returned from a retracted position to a sealing position. Consequently, liquid-tightness of a first flow path 52 and a second flow path 102 is maintained, and, therefore, a liquid medical agent in these flow paths is securely prevented from leaking out of the connector assembly 1.

Incidentally, while the number of the grooves 48 and the projections 14 formed is four in the present embodiment, this number is not limitative. For example, the number may be one, two, three, or five or more.

### <Fourth Embodiment>

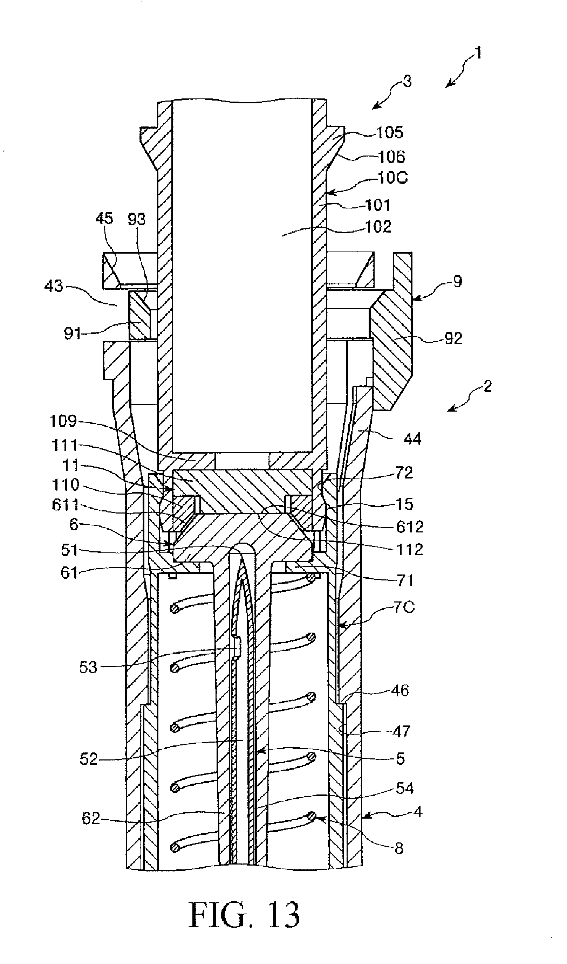

FIG. 13 is a longitudinal sectional view illustrating a first connector and a second connector in a connector assembly (fourth embodiment) according to the present invention.

Now, the fourth embodiment of the connector assembly according to the present invention will be described below, referring to the figure. The following description will be centered on the difference of this embodiment from the above-described embodiments, and descriptions of the same items as above will be omitted.

This embodiment is the same as the above-described first embodiment, except for differences in the constitutions of the first connector body and the second connector body.

As shown in FIG. 13, an inner tube 7C of the first connector 2 is provided at an inner peripheral portion of the distal end thereof with an inner tube side engaging part 72 composed of a reduced diameter part reduced in inside diameter. In addition, a second connector body 10C is provided at an outer peripheral portion of the proximal end thereof with a second connector body side engaging part 15 composed of an enlarged diameter part enlarged in outside diameter. In an assembled state, the inner tube side engaging part 72 and the second connector body side engaging part 15 can be engaged with each other. Further, in this instance, a head part 61 of a first sealing member 6 and a second sealing member 11 are compressed in the directions for approaching each other. This ensures that a distal end face 612 of the head part 61 of the first sealing member 6 and a proximal end face 112 of the second sealing member 11 are put into close contact with each other more assuredly.

In addition, the engaged state of the inner tube side engaging part 72 of the inner tube 7C of the first connector 2 and the second connector body side engaging part 15 of the second connector body 10C is maintained until the first sealing member 6 is located in a sealing position and the second connector 3 is pulled relative to the first connector 2 with a force on such a level that the engagement between these engaging parts is released. Thus, the inner tube side engaging part 72 of the inner tube 7C of the first connector 2 and the second connector body side engaging part 15 of the second connector body 10C functions as close-contact maintaining means, like the fitting member 12 in the first embodiment described above.

### <Fifth Embodiment>

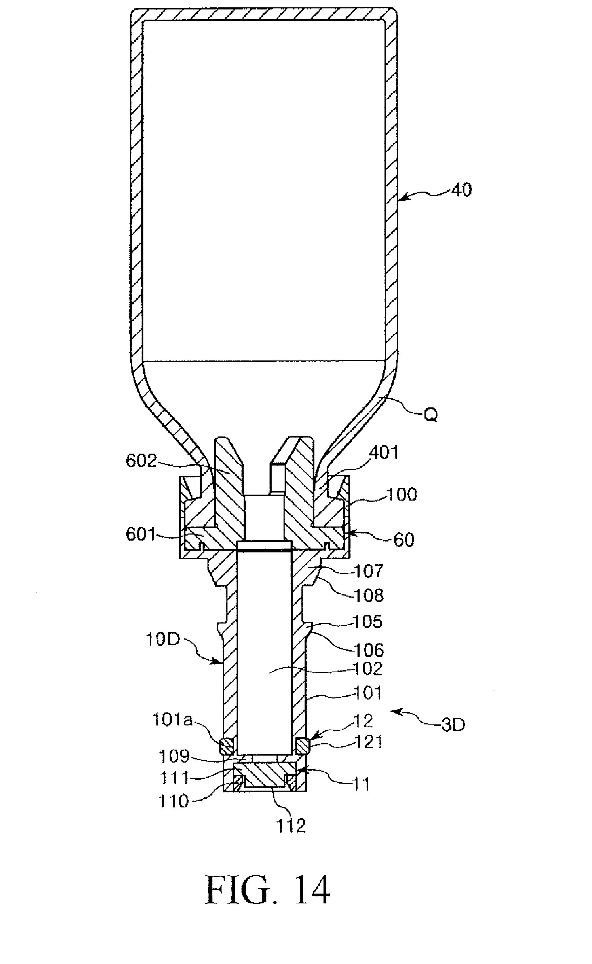

FIG. 14 is a longitudinal sectional view illustrating a second connector in a connector assembly (fifth embodiment) according to the present invention.

Now, the fifth embodiment of a connector assembly according to the present invention will be described below, referring to the figure. The following description will be centered on the difference of this embodiment from the above-described embodiments, and descriptions of the same items as above will be omitted.

This embodiment is the same as the above-described first embodiment, except for a difference in constitution/shape of the second connector.

A second connector 3D shown in FIG. 14 is mounted to a mouth part 401 of a vial (liquid container) 40 capable of containing a dissolving liquid P for dissolving a medical agent Q. The vial 40 is a member which is in the shape of a bottomed cylinder and has a distal end opening part which opens at the distal end thereof. The distal end opening part constitutes the mouth part 401 of the vial 40.

In the second connector 3D, a distal end portion of a second connector body 10D is enlarged in diameter, and the distal end portion is, as a connecting part 100, connected to the mouth part 401 of the vial 40 by fitting.

In addition, an adapter 60 for making securer the connection with the connecting part 100 of the second connector 3D is mounted to the mouth part 401 of the vial 40. The adapter 60 is a tubular member, which is provided with a flange part 601 projectingly formed at an outer peripheral portion of the proximal end thereof. Of the adapter 60, a distal end portion 602 is inserted into the mouth part 401 of the vial 40, and the flange part 601 is clamped

(compressed) between the mouth part 401 and the connecting part 100 of the second connector 3D.

By the second connector 3D constituted as above, the connector assembly 1 in the assembled state can supply the dissolving liquid P from a syringe 30 into the vial 40.

### <Sixth Embodiment>

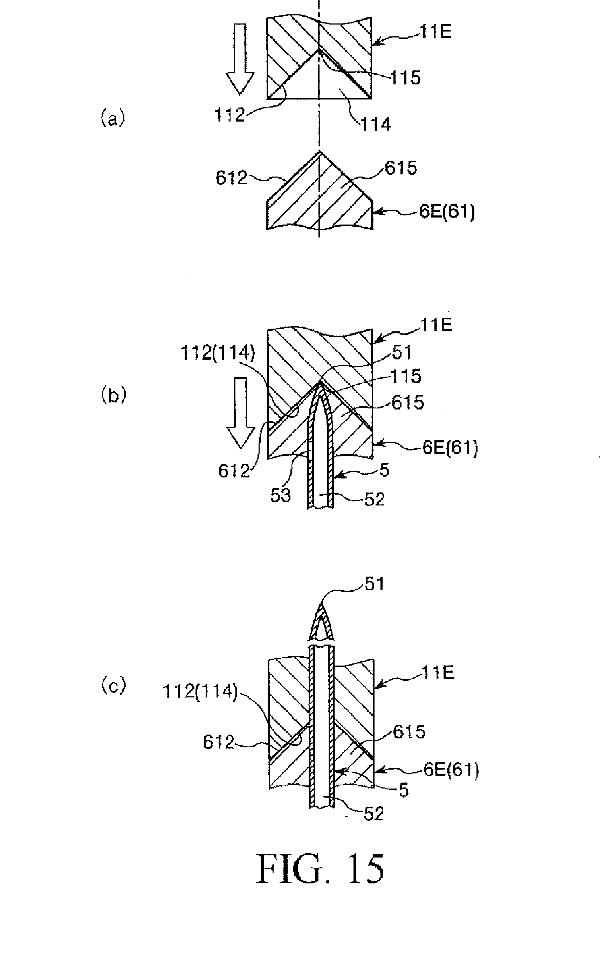

FIG. 15 shows longitudinal sectional views illustrating a process until a first sealing member and a second sealing member in a connector assembly (sixth embodiment) according to the present invention are brought into close contact with each other.

Now, the sixth embodiment of the connector assembly according to the invention will be described, referring to the figure. The following description will be centered on the difference of this embodiment from the above-described embodiments, and descriptions of the same items as above will be omitted.

This embodiment is the same as the above-described first embodiment, except for differences in the shapes of the first sealing member and the second sealing member.

As shown in (a) to (c) of FIG. 15, a first sealing member 6E has, at a first to-be-pierced part 61 thereof, a protuberance 615 which is protuberant in a conical shape and is formed projectingly in the distal direction. In addition, a second sealing member 11E is formed with a recess 114.

The recess 114 is hollowed in a conical shape. As shown in (c) of FIG. 15, in an assembled state, the protuberance 615 of the first sealing member 6E is inserted into the recess 114 of the second sealing member 11E. Further, in this instance, a distal end face 612 of the first sealing member 6E (the protuberance 615) and a proximal end face 112 of the second sealing member 11E (the recess 114) make close contact with each other. In addition, the area of close contact between these surfaces is greater than the area of close contact between the distal end face 612 of the first sealing member 6 and the proximal end face 112 of the second sealing member 11 in the first embodiment described above. This ensures that the liquid-tightness (gas-tightness) of a first flow path 52 and a second flow path 102, particularly at the joint between these flow paths, can be maintained more assuredly and that the dissolving liquid P passing through these flow paths can be securely prevented from leaking out of the connector assembly 1 in the assembled state.

Further, as shown in (b) of FIG. 15, when a hollow needle 5 pierces the second sealing member 11E, a needle point 51 of the hollow needle 5 is guided to a center (bottom portion) 115 of the recess 114 (is centered). This enables the hollow needle 5 to pierce the center of the second sealing member 11E.

### <Seventh Embodiment>

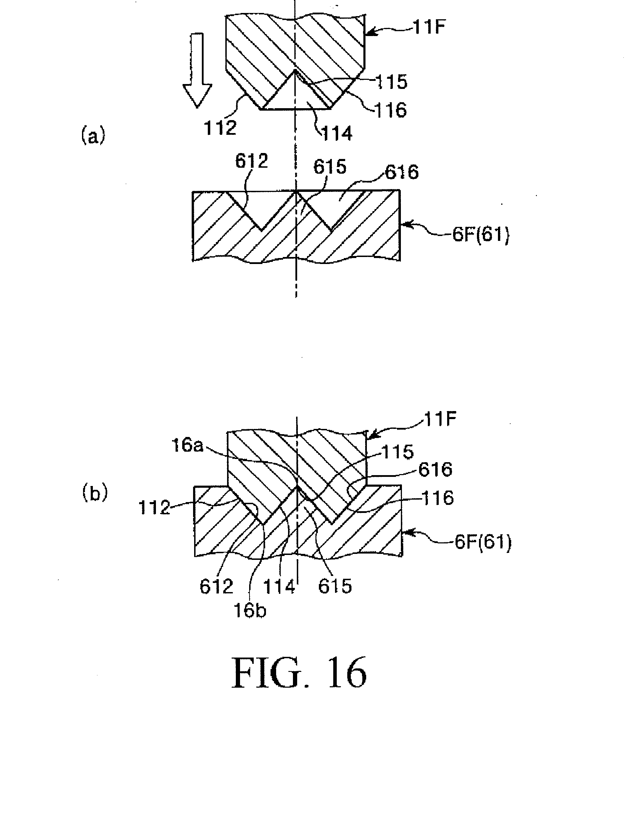

FIG. 16 shows longitudinal sectional views illustrating a process until a first sealing member and a second sealing member in a connector assembly (seventh embodiment) according to the present invention are brought into close contact with each other.

Now, the seventh embodiment of the connector assembly according to the invention will be described below, referring to the figure. The following description will be centered on the difference of this embodiment from the above-described embodiments, and descriptions of the same items as above will be omitted.

This embodiment is the same as the above-described sixth embodiment, except for differences in the shapes of the first sealing member and the second sealing member.

As shown in (a) and (b) of FIG. 16, a first to-be-pierced part 61 of a first sealing member 6F is formed with a ring-shaped recess 616 on the outer periphery side of a protuberance 615. The recess 616 is a part which is triangular in sectional shape. In addition, a recess 114 in a second sealing member 11F is formed with a tapered part 116 where the outside diameter of an outer peripheral portion gradually decreases in the proximal direction. In other words, an outside portion of a proximal end face 112 of the second sealing member 11F is in a tapered shape.

As shown in (b) of FIG. 16, in the assembled state, the distal end face 612 of the first sealing member 6F and the proximal end face 112 of the second sealing member 11F make close contact with each other. Further, in the assembled state, even if the close contact at a close contact point 16a is unsatisfactory, close contact can be securely achieved at another close contact point 16b. This ensures that the liquid-tightness (gas-tightness) of a first flow path 52 and a second flow path 102, particularly at the joint of these flow paths, can be maintained more assuredly and that a dissolving liquid P passing through the flow paths is securely prevented from leaking out of the connector assembly 1 in the assembled state.

### <Eighth Embodiment>

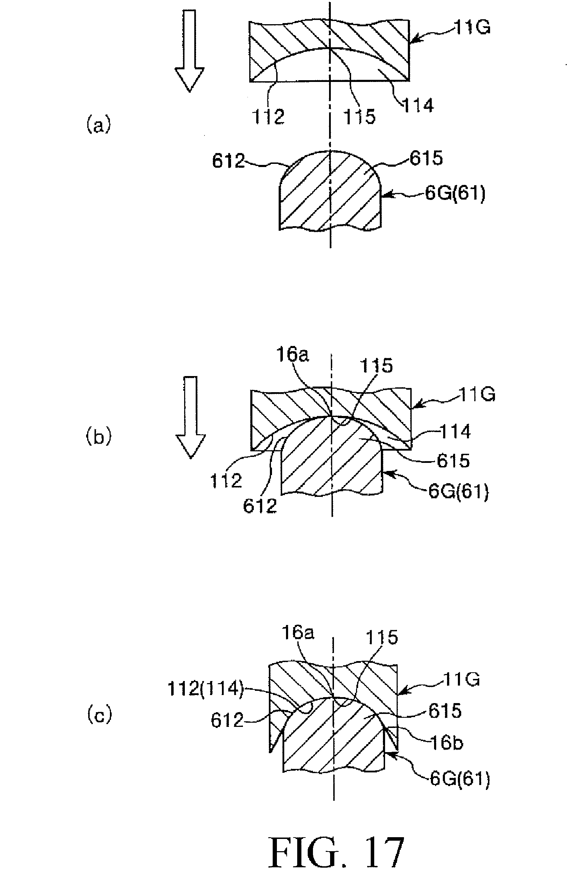

FIG. 17 shows longitudinal sectional views illustrating a process until a first sealing member and a second sealing member in a connector assembly (eighth embodiment) according to the present invention are brought into close contact with each other.

Now, the eighth embodiment of the connector assembly according to the invention will be described below, referring to the figure. The following description will be centered on the difference of this embodiment from the above-described embodiments, and descriptions of the same items as above will be omitted.

This embodiment is the same as the above-described sixth embodiment, except for differences in the shapes of the first sealing member and the second sealing member.

As shown in (a) to (c) of FIG. 17, of a first sealing member 6G, a distal end face 612 of a protuberance 615 is spherical in shape. In addition, of a second sealing member 11G, a proximal end face 112 of a recess 114 is spherical in shape. As shown in (a) of FIG. 17, in a natural condition where no external force is exerted, the distal end face 612 of the protuberance 615 and the proximal end face 112 of the recess 114 are different from each other in curvature; specifically, the curvature of the distal end face 612 of the protuberance 615 is greater than the curvature of the proximal end face 112 of the recess 114. This ensures that, as shown in (c) of FIG. 17, in an assembled state, the proximal end face 112 of the recess 114 adapts itself to the shape of the distal end face 612 of the protuberance 615, in other words, it is deformed so that its curvature becomes equal to the curvature of the distal end face 612 of the protuberance 615. This results in that the close contact force between the distal end face 612 of the protuberance 615 and the proximal end face 112 of the recess 114 is maximized at a close contact point 16a, and the close contact force decreases gradually from the close contact point 16a toward a close contact point 16b. In the case where it is desired to vary the close contact force in this manner, the constitution of the present embodiment is effective.

While the connector assembly according to the present invention has been described above referring to the embodiments shown in the drawings, the invention is not limited to the embodiments, and each of the components of the connector assembly may be replaced by an arbitrarily constituted one that can exhibit the same function. Further, arbitrary structures may be added.

In addition, the connector assembly according to the invention may be a combination of arbitrary two or more constitutions (features) of the above-described embodiments.

Further, in the first embodiment above, the fitting member mounted to the second connector may be omitted.

In addition, in the second embodiment above, the ribs formed in the second connector may be omitted.

Further, in the third embodiment above, the grooves formed in the first connector and the projections formed in the second connector may be omitted.

In addition, in the fourth embodiment above, the inner tube engaging part formed in the first connector and the second connector body side engaging part formed in the second connector may be omitted.

Further, the locking means for connecting the first connector and the second connector to each other in the assembled state may be locking means composed of a groove which is formed in a wall part (tubular wall) of one of the first connector body and the second connector body and a projection which is formed in a wall part of the other and which is inserted into the groove in the assembled state.

### Industrial Applicability

The connector assembly according to the present invention includes: a first connector equipped with a first connector body tubular in shape, a hollow needle supported on an inner side of the first connector body and having an opening part opening at a distal end portion thereof, and a first sealing member formed of an elastic material and having a first to-be-pierced part which can be pierced through by the hollow needle;
a second connector equipped with a second connector body tubular in shape, and a second sealing member formed of an elastic material and having a second to-be-pierced part which, in a mounted state of being inserted in the first connector, is pierced through by the hollow needle; locking means for connecting the first connector and the second connector to each other in the mounted state; unlocking means for releasing connection of the first connector and the second connector by the locking means; and close-contact maintaining means which, in the mounted state, maintains close contact between the first to-be-pierced part and the second to-be-pierced part; wherein in the mounted state, the first to-be-pierced part and the second to-be-pierced part are pierced through by the hollow needle while being in close contact with each other, and the opening part of the hollow needle is located on a distal end side relative to the second to-be-pierced part and exposed to an inside of the second connector body, and a lumen of the hollow needle and a lumen of the second connector body communicate with each other through the opening part; and when the second connector is pulled out of the first connector, the close contact between the first to-be-pierced part and the second to-be-pierced part is maintained by the close-contact maintaining means until the opening part of the hollow needle comes to be located on a proximal end side relative to the second to-be-pierced part. Therefore, a liquid can be transferred safely and assuredly from the first connector side to the second connector side or in the reverse direction. Accordingly, the connector assembly of the present invention has an industrial applicability.

## Claims

1. A connector assembly comprising:
a first connector equipped with a first connector body tubular in shape, a hollow needle supported on an inner side of the first connector body and having an opening part opening at a distal end portion thereof, and a first sealing member formed of an elastic material and having a first to-be-pierced part which can be pierced through by the hollow needle;
a second connector equipped with a second connector body tubular in shape, and a second sealing member formed of an elastic material and having a second to-be-pierced part which, in a mounted state of being inserted in the first connector, is pierced through by the hollow needle;
locking means for connecting the first connector and the second connector to each other in the mounted state;
unlocking means for releasing connection of the first connector and the second connector by the locking means; and
close-contact maintaining means which, in the mounted state, maintains close contact between the first to-be-pierced part and the second to-be-pierced part;
wherein in the mounted state, the first to-be-pierced part and the second to-be-pierced part are pierced through by the hollow needle while being in close contact with each other, the opening part of the hollow needle is located on a distal end side relative to the second to-be-pierced part and exposed to an inside of the second connector body, and a lumen of the hollow needle and a lumen of the second connector body communicate with each other through the opening part; and
when the second connector is pulled out of the first connector, the close contact between the first to-be-pierced part and the second to-be-pierced part is maintained by the close-contact maintaining means until the opening part of the hollow needle comes to be located on a proximal end side relative to the second to-be-pierced part.

2. The connector assembly according to claim 1, wherein the close-contact maintaining means has a biasing member which is disposed inside the first connector body and which biases the first to-be-pierced part in a distal direction.

3. The connector assembly according to claim 2, wherein a sliding resistance generated between the hollow needle and the first to-be-pierced part when the second connector is pulled out of the first connector is smaller than a biasing force of the biasing member.

4. The connector assembly according to claim 2, wherein the close-contact maintaining means has a fitting member being ring-like in shape, being disposed at an outer peripheral portion of the second connector body and, in the mounted state, being fitted to an inner peripheral portion of the first connector body.

5. The connector assembly according to claim 4, wherein a sliding resistance generated between the inner peripheral portion of the first connector body and the fitting member when the second connector is pulled out of the first connector is greater than the biasing force of the biasing member.

6. The connector assembly according to claim 2, wherein the close-contact maintaining means has a plurality of ribs which are formed at an outer peripheral portion of the second connector body in a longitudinal direction thereof and which, in the mounted state, make contact with an inner peripheral portion of the first connector body.

7. The connector assembly according to claim 6, wherein a sliding resistance generated between the inner peripheral portion of the first connector body and the plurality of ribs when the second connector is pulled out of the first connector is greater than the biasing force of the biasing member.

8. The connector assembly according to claim 2, wherein the close-contact maintaining means has a spiral groove formed in an inner peripheral portion of the first connector body, and a projection which is projectingly formed at an outer peripheral portion of the second connector body and which, in the mounted state, is inserted in the groove.

9. The connector assembly according to claim 2,
wherein an inner tube movable along an axial direction of the first connector body is disposed on the inner side of the first connector body; and
the close-contact maintaining means has an inner tube side engaging part provided at a distal end portion of the inner tube, and a second connector body side engaging part which is provided at an end portion of the second connector body and which, in the mounted state, is engaged with the inner tube side engaging part so as to compress the first to-be-pierced part and the second to-be-pierced part toward each other.

10. The connector assembly according to claim 1, wherein the locking means comprises a first engaging part disposed on the first connector body so as to be movable in a radial direction of the first connector body, an elastic piece which is provided in the first connector body and which biases the first engaging part toward the inner side of the first connector body, and a second engaging part which is provided in the second connector body and which is engaged with the first engaging part.
